(19)

Europäisches Patentamt

European Patent Office

Office européen des brevets

(11)  **EP 4 236 861 B1**

(12)  **EUROPEAN PATENT SPECIFICATION**

(45) Date of publication and mention
of the grant of the patent:
**30.04.2025  Bulletin 2025/18**

(21) Application number: **21801931.3**

(22) Date of filing: **01.11.2021**

(51) International Patent Classification (IPC):
*A61C 8/00* (2006.01)     *A61L 27/28* (2006.01)

(52) Cooperative Patent Classification (CPC):
**A61C 8/0013; A61L 27/10;** A61C 2008/0046;
A61L 2430/12

(86) International application number:
**PCT/EP2021/080246**

(87) International publication number:
**WO 2022/090530 (05.05.2022 Gazette 2022/18)**

(54) **DENTAL IMPLANT WITH PROTECTIVE LAYER AND METHOD OF PREPARING SAID DENTAL IMPLANT**

ZAHNIMPLANTAT MIT SCHUTZSCHICHT UND VERFAHREN ZUR HERSTELLUNG DES ZAHNIMPLANTATS

IMPLANT DENTAIRE AVEC COUCHE PROTECTRICE ET PROCÉDÉ DE PRÉPARATION DUDIT IMPLANT DENTAIRE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **02.11.2020  EP 20205274**

(43) Date of publication of application:
**06.09.2023  Bulletin 2023/36**

(73) Proprietor: **Institut Straumann AG**
**4002 Basel (CH)**

(72) Inventors:
• **ALARCON, Marta Girona**
**79651 Lörrach (DE)**
• **ARMUTLULU, Andac**
**4002 Basel (CH)**

(74) Representative: **Schaad, Balass, Menzl & Partner**
**AG**
**Bellerivestrasse 20**
**Postfach**
**8034 Zürich (CH)**

(56) References cited:
WO-A1-2018/189185     US-A1- 2007 202 144
US-A1- 2015 367 043

• CAMPBELL DUNCAN I. ET AL: "The Effect of a
Keratin Hydrogel Coating on Osseointegration:
An Histological Comparison of Coated and Non-
coated Dental Titanium Implants in an Ovine
Model", vol. 13, no. 2, 1 February 2013
(2013-02-01), India, pages 159 - 164,
XP055783968, ISSN: 0972-8279, Retrieved from
the Internet <URL:http://link.springer.com/
article/10.1007/s12663-013-0482-y/fulltext.html>
[retrieved on 20210310], DOI: 10.1007/
s12663-013-0482-y

• BAJESTANI MARYAM IJADI ET AL: "Material
properties and cell compatibility of
poly([gamma]-glutamic acid)-keratin hydrogels",
INTERNATIONAL JOURNAL OF BIOLOGICAL
MACROMOLECULES, ELSEVIER BV, NL, vol.
142, 14 October 2019 (2019-10-14), pages 790 -
802, XP086032475, ISSN: 0141-8130, [retrieved
on 20191014], DOI: 10.1016/
J.IJBIOMAC.2019.10.020

EP 4 236 861 B1

**(Cont. next page)**

- **ALINA SIONKOWSKA ED - MÜLLER ALEJANDRO J ET AL: "Current research on the blends of natural and synthetic polymers as new biomaterials: Review", PROGRESS IN POLYMER SCIENCE, PERGAMON PRESS, OXFORD, GB, vol. 36, no. 9, 4 May 2011 (2011-05-04), pages 1254 - 1276, XP028238729, ISSN: 0079-6700, [retrieved on 20110525], DOI: 10.1016/ J.PROGPOLYMSCI.2011.05.003**

**Description**

The present invention relates to a dental implant being at least partially covered with a protective layer.

Dental implants are used to replace individual teeth or for anchoring more complex structures, which generally replace several or even all teeth. Implants have two essential parts: an anchoring part and an abutment part. The anchoring part is embedded in the bone, where it osseointegrates with the bone tissue to provide a firm anchor for the prosthesis. The abutment extends into the oral cavity and provides a support for the prosthesis. The desired prosthetic element (e.g. bridge or crown) is fastened over the abutment such that at least part of the abutment is housed within the prosthesis and provides core support to this. The prosthetic element can be adhesively bonded, cemented, screwed or directly veneered onto the abutment.

Implants, such as dental implants, are well known in the art. They generally consist of a material, which is biocompatible and which additionally has favorable mechanical properties.

In addition, it is required that the dental implant provides good osseointegration. The term "osseointegration" designates the direct structural and functional connection between living bone and the surface of the load-bearing implant. A good osseointegration means that the implant, after reaching a primary stability by screwing it into the bone, safely ossifies within a short healing time so that a permanent bond between implant and bone is obtained.

In the beginning phase of modern implantology a minimally rough surface was the gold standard. Later, an increase to moderately rough surfaces led to faster and firmer osseointegration in several experimental studies using various animal models.

A breakthrough technology in the development of highly osseointegrative dental implants is the so-called "SLA" process which is disclosed in EP 0 388 576, involving sandblasting the implant's surface followed by acid-etching to achieve an optimal topography for the attachment of bone cells.

Based on the "SLA" technology, the so-called "SLActive" surface was developed and disclosed in WO 00/44305, which further comprises conditioning the "SLA" surface either in nitrogen or in an isotonic saline solution, thereby maintaining the high hydrophilicity of the "SLA" surface which would otherwise be lost due to reaction with the atmosphere. The packaging process is time-consuming and expensive, which is a disadvantage of this procedure.

US 2014/172028 discloses a surface treatment process of an endosseous implantable medical device by covering the surface of the medical implant with a sugar or a sugar alcohol.

WO 2018/189185 discloses a dental implant made of a ceramic material. The implant surface has at least partially a contact angle of less than 20° and the implant surface is at least partially covered with a protective layer. Said protective layer comprises a water-soluble dextran having a molecular weight of more than 15' 000 Da.

WO 2008 /098976 discloses a process for the production of implants with a hydrophilic surface by covering the surface of said implant with a salty layer.

US 2009/0132048 discloses a dental implant which is at least partly provided with a protective layer which dissolves on contact with body fluid and/or the bone, said protective layer consisting of salt. However, salty protective layers, in particular those consisting of NaCl, show a reduced long-term stability.

WO 03/030957 discloses an implant having roughened hydroxylated and hydrophilic surface and being treated in the hydroxylated state with high-energy ultraviolet radiation. One disadvantage of this solution is the additional treatment step which is supposed to be carried out by the surgeon in particular.

Campbell et al "The Effect of a Keratin Hydrogel Coating on Osseointegration: A Histological Comparison of Coated and Non-coated Dental Titanium Implants in an Ovine Model", JOURNAL OF MAXILLOFACIAL AND ORAL SURGERY vol. 13, no. 2 1 February 2013 (2013-02-01), pages 159-164, disclose the effect of a keratin hydrogel on osseointegration.

Bajestani et al ("Material properties and cell compatibility of poly ([gamma]-glutamic acid)-keratin hydrogels", INTERNATIONAL JOURNAL OF BIOLOGICAL MACROMOLECULES, ELSEVIER BV, NL, vol. 142, 14 October 2019 (2019-10-14), pages 790-802) disclose the preparation of gamma-PGA-Keratin hydrogels.

Sionkowska et al ("Current research on the blends of natural and synthetic polymers as new biomaterials: Review", PROGRESS IN POLYMER SCIENCE, PERGAMON PRESS, OXFORD, GB, vol. 36, no. 9, 4 May 2011 (2011-05-04), pages 1254-1276) disclose a study of hydrolized keratin for making biofilms.

US 2015/367043 A1 discloses methods of making charged keratins by separating one from the other, e.g., by chromatography, and optionally further processing or purifying the retained fraction or fractions.

US 2007/202144 A1 shows a method of coating of an implant with a dry protective layer comprising protein, peptide, polysaccharide or a glycolipid or a small molecule.

The object of the present invention was to provide a protective layer for dental implants which prevents the surface from turning to hydrophobic. In addition, the protective layer needs to be biocompatible and the product should not suffer from hydrothermal aging while being hydrophilic.

The problem is solved by a dental implant according to claim 1 and a method for preparing said dental implant according to claim 11.

Further preferred embodiments are subject of the dependent claims.

It was found that a dental implant comprising an implant surface having at least partially a contact angle of less than 20°, said implant surface being at least partially covered by a protective layer comprising a keratin hydrolyzate provides an excellent osteointegration. The protective layer has a water content of less than 10% by weight. Interestingly, it is no longer necessary that the dentist removes the protective layer before implanting the dental implant. Further, the protective layer ensures hydrophilicity until the contact of the implant with the bone is established. Due to its composition, the protective layer according to the present invention is biocompatible and has a good accessibility.

The protective layer according to the present invention prevents the deposition of contaminants on the implant surface having at least partly a contact angle of less than 20°. In addition, the contact angle on the surface of the protective layer is less than 20°, that is, even the protective layer itself has an excellent wettability which allows an accelerated osseointegration even without removing the protective layer. In particular, it could be shown that the protective layer according to the present invention can withstand harsh conditions such as high humidity, low temperature or low pressure. The protective layer of the dental implant according to the present invention allows to maintain the hydrophilicity of the surface during the storage time in the air.

The standard parameter to determine the hydrophilicity of a surface is the measurement of the water contact angle (DIN 55660-2). The contact angle quantifies the wettability of the implant by water and therefore the degree of contact with the hydrophilic surroundings. The term "contact angle" as used in the context of the present invention relates to the contact angle of water on the surface, i.e. to the angle formed at the interface where water meets the surface. Thereby, the term "water" used in the context of the contact angle measurement the context of the present invention relates to the contact angle of water on the surface, i.e. to the angle formed at the interface where water meets the surface. Thereby, the term "water" used in the context of the contact angle measurement relates to pure water, specifically ultrapure water. In particular, the contact angle measurement is carried out by the sessile drop method (e.g. by means of a device of the type EasyDrop DSA20E, Kruss GmbH) using a drop size of 0.1 µL for hydrophilic and 0.3 µL for hydrophobic discs of 5 mm diameter, meaning that keratin coated discs are measured with 0.1 uL droplet. Contact angles were calculated by fitting a circular segment function to the contour of the droplet placed on the surface. The terms "hydrophilic" or "hydrophilicity" as used in the context of the present invention refer to a water contact angle of the hydrophilic surface area directly on the dental implant being less than 20°, more preferably less than 10°.

Such a hydrophilic surface having a contact angle of less than 20° can be obtained for example by the so-called SLA technology or by other etching procedures.

The expression "keratin hydrolyzate" stands for a hydrolyzate obtained from keratin wherein the peptide chains were divided into smaller peptides with lower molecular weights, that is a molecular weight of less than 20'000 g/mol. The molecular weight of the keratin hydrolysate may be determined for example by SDS-PAGE or by measuring absorption at 280 nm. A keratin hydrolyzate can be prepared, for example, by an acid or alkaline hydrolysis, or by enzymatic digestion. The keratin source usually used may be from several origins such as, for example, human hair fibers, wool, animal hair, feathers and horns.

As the keratin hydrolyzate exists in solution as colloids, it is capable of interacting with the blood and thus facilitates the protein absorption and the formation of a layer of blood components on the implant surface.

Preferably, the keratin hydrolyzate has a mean molecular weight of less than 20'000 Da, preferably less than 10'000 Da, most preferably less than 5'000 Da. It has to be assumed that such keratin hydrolyzates lead to very dense, well-structured protective layers.

The molecular weight of the keratin hydrolyzate can be determined, for example, by SDS-PAGE method on tricine polyacrylamide gels in minicell Novex Model-3540 (USA) with constant voltage 125 V and current intensity 80 mA on start and 40 mA on end (Krejci et al, preparation and characterization of keratin hydrolyzates, Mathematical Methods and Techniques in Engineering and Environmental Science, ISBN: 978-1-61804-046-6).

Preferably, the protective layer has a thickness of 30 to 200 nm, most preferably of 30 to 90 nm. Interestingly, such thin layers can protect the hydrophilicity of the dental implant, but the roughness of the dental implant is maintained on the surface of the protective layer and results therefore in a better adherence. Therefore, a dental implant covered with a protective layer according to the present invention shows essentially the same surface roughness as a dental implant without said protective layer.

In fact, the density of the protective layer according to the present invention is remarkable. For both, a protective layer consisting of keratin hydrolyzate only and for one comprising keratin hydrolyzate together with a salt, the density of said layers is about 5% higher than a corresponding protective layer comprising glucose and a salt.

In one embodiment of the present invention, the protective layer additionally comprises a salt, preferably a bivalent salt, and most preferably $MgCl_2$.

In a further embodiment of the present invention, the protective layer essentially free from any salts. Such a layer showed an outstanding homogeneity and was essentially free of cracks. In addition, the microroughness of the protective layer of the present invention is comparable with the microroughness of a non-coated dental implant. In fact, it is even difficult to distinguish between samples coated with the protective layer and the uncoated samples by scanning electron microscopy (SEM).

Furthermore, it was shown that the protective layer according to the present invention avoids the hydrothermal aging and keeps the hydrophilicity of the implant surface over time, preferably for at least one year, in particular of Y-TZP ceramic material.

Preferably, the dental implant comprising the protective layer according to the present invention is sterilized by ethylene oxide gas. This technique can be applied on the dental implant that is packed already. The ethylene oxide gas permeates the packaging and reaches the dental article to be sterilized. It could be shown that a sterilization by ethylene oxide gas does not negatively influence the stability of the protective layer. The hydrophilicity of the surface remains stable, which is particularly relevant when the dental implant is stored over relative long periods. In particular, when performing climatic stress over 8 weeks, the protective layer according to the present invention showed significant better hydrophilicity in comparison with a protective layer comprising, for example, agarose. Even after ethylene oxide (EO) sterilization, the implant surface coated by a protective layer according to the present invention was highly attractive for blood. Unprotected implants did not show the same affinity for blood which is in agreement with their hydrophobic nature.

Preferably, the protective layer is free of hydroxyapatite since protective layers that are free from hydroxyapatite have a higher density than the one comprising a combination of keratin hydrolyzate and hydroxyapatite.

Preferably, the dental implant is made of ceramic, preferably of an yttria stabilized zirconia ceramic. One of the main advantages of a zirconia implant is its natural white color that makes it undistinguishable from natural teeth. In addition, zirconia implants trigger a higher fibrinogen adsorption and a lower risk of bone loss. The protective layer shows similar features on a ceramic dental implant compared to an uncoated dental implant with the exception of a small decrease in the sharpness of the image, that is, there are smoother edges in the topography.

Alternatively, the dental implant is made of metal, preferably of titanium or a titanium alloy. A preferred titanium alloy is a binary titanium-zirconium alloy, and most preferably a binary titanium-zirconium alloy containing from 13 to 17 wt-%, more preferably from 13 to 15 wt-% of zirconium. This material has been shown to exhibit outstanding properties regarding mechanical stability and biofunctionality; a particularly preferred binary titanium-zirconium containing zirconium in the above-mentioned ranges is available under the trade-name Roxolid® (Institut Straumann AG, Switzerland). It was shown that the protective layer accroding to the present invention can also maintain the hydrophilicity of such an implant until the contact of the implant with the bone is established.

The dental implant according to the present invention can be constructed in one or more parts, in which case they consist of at least an anchoring part, often referred to in isolation as the implant, and an abutment, sometimes referred to as a spacer or post element. The anchoring part is usually either embedded completely in the bone, that is to say to the height of the alveolar crest (so called bone level implants), or protrudes by a few millimetres from the alveolar crest into the soft tissue (so called soft tissue level implants). The abutment is mounted either directly or indirectly to the anchoring part after the latter has become incorporated (osseointegrated) into the bone, or directly after the anchoring part has been inserted. It can also be attached to the anchoring part prior to insertion.

Preferably, such an anchoring part of a multi-part dental implant is generally in a cylindrical or conical shape, having an apical end with a body portion and a coronal end with a neck portion intended to receive an abutment in case of multi-part implants, and a transition portion being arranged in axial direction between the body portion and the neck portion. On said abutment a crown, a bridge or another suprastructure may be fixed, for example by screwing, cementing or gluing. The body portion is intended to be directed against the bone tissue in the implanted state and the neck portion is intended to direct against the soft tissue, whereas the transition portion may direct against the bone tissue or the soft tissue depending on the patient. Preferably, the total length of the anchoring part is 4 to 19 mm in the axial direction. The body portion with the apical end is preferably 50 to 90% of the total length of the implant in axial direction. The neck portion with the coronal end which is essentially be intended to be in contact with the soft tissue covers preferably 10 to 40% of the total length of the implant in axial direction. The transition portion covers preferably 0 to 40% of the total length of the implant in axial direction. The neck portion has preferably a length of 1 to 4 mm, most preferably 1.8 to 2.8 mm, the transition portion has preferably a length of 0 to 2 mm, most preferably 1 to 2 mm, and the body portion has preferably a length of 4 to 18 mm.

The body portion typically includes a threaded part which may be self-tapping or non-self-tapping.

Similar to the multi-part implant system, the anchoring portion of such a monotype implant is generally in a cylindrical or conical shape, having an apical end with a body portion and a coronal end with a neck portion passing into the abutment. The anchoring portion is typically provided with a threaded part, which may be self-tapping or non-self-tapping. A transition portion is arranged in axial direction between the body portion and the neck portion. On the abutment portion, a crown, a bridge or another suprastructure may be fixed, for example, by screwing, cementing or gluing. The body portion is intended to be directed against the bone tissue in the implanted state and the neck portion is intended to direct against the soft tissue, whereas the transition portion may direct against the bone tissue or the soft tissue depending on the patient.

The protective layer according to the present invention covers at least partially, preferably fully the hydrophilic surface of the dental implant and is preferably a continuous, crackless layer. In particular, if not only the body portion, but also the transition portion or the transition portion and the neck portion are covered with the protective layer, said protective layer is preferably one continuous layer.

In order to obtain an excellent osseointegration, the body portion is typically provided with a surface roughness. The term "surface roughness" within the context of the present invention stands for "arithmetical mean height" (Sa). Specifically, the surface roughness Sa (the arithmetic mean deviation of the surface in three dimensions) is determined in analogy to EN ISO 4287 relating to the respective parameters Ra in two dimensions. For the parameters in three dimensions, it is further referred to ISO 25178 (Sa is defined as Sq). Preferably, the surface roughness (Sa) is between 2 and 10 $\mu$m, preferably between 2 and 5 $\mu$m. Surprisingly, it is possible to maintain the surface roughness by the protective layer according to the present invention since the layer is extremely thin.

Preferably, the protective layer has a water content of less than 1% by weight. The absence of water to obtain a stable protective layer.

According to a further embodiment, the body portion is provided with a machined, but hydrophilic surface. Thus, said dental implants are acid etched, but no roughening step such as sandblasting, was applied.

The dental implant coated with the protective layer is prepared by a comprising at least the following steps:

a) providing the surface of the dental implant with a contact angle of less than 20°

b) covering at least partly the surface of the dental implant having a contact angle of less than 20° with a solution or suspension comprising the keratin hydrolyzate

c) drying the dental implant in order to obtain a protective layer.

In order to providing the surface of the dental implant with a contact angle of less than 20°, the dental implant is preferably etchend by an inorganic acid or a blend of inorganic acids. More preferably, the inorganic acid(s) is/are selected from the group consisting of hydrofluoric acid, hydrochloric acid, sulphuric acid or mixtures thereof. Before carrying out the implant can at least partially be roughend mechanically and/or by using plasma technology and/or by laser structuring or by other methods known to the skilled person.

A preferred surface topography of an implant according to the present invention can be obtained for example by applying the process described in EP 1 982 670 or EP 1 982 671 before coating the roughened and etched surface.

Preferably, the surface of the dental implant is at least partly covered by dipping the implant into an aqueous solution or suspension comprising the keratin hydrolyzate. However, other methods to apply the aqueous solution to the surface to be protected are possible as well. Said dipping procedure ensures a constant thickness of the protective coating.

Preferably, the drying in step c), that is, the removal of water, is carried out by microwave treatment or by clear air drying (no specific drying, but storage at room temperature in a clean environment) drying in a convection oven, a vacuum oven or in an airstream, said airstream preferably having a temperature of 20°C to 80°C, most preferably of 30°C to 40°C. Preferably, the dental implants according to the present invention are dried in a convection oven at 20 to 80°C, preferably at 70°C, for 10 to 120 minutes, preferably for 15 to 90 minutes, most preferably for 20 to 60 minutes, ensuring that the protective layer is dried out completely. The above drying method does not induce splatters of the coating substance on the sample and allows to avoid hydrothermal aging.

Preferably, the hydrolyzed keratin is solubilized in an aqueous solution or aqueous suspension in a concentration of 0.1 to 10% by weight to volume (w/v), more preferably 1 to 5% by weight to volume (w/v).

**Figures**

The present invention is further illustrated by the following figures and examples:

Figure 1 refers to a first embodiment of the present invention;

Figure 2 refers to a second embodiment of the present invention;

Figure 3 refers to a third embodiment of the present invention;

Figure 4 refers to a forth embodiment of the present invention;

Figure 5 refers to a fifth embodiment of the present invention;

Figures 6a to 6c refer to static contact angle for different protective layers with different synthetic parameters for the coating procedure. Fig. 6a shows SCA (static contact angle) changing the coating time. Fig. 6b shows SCA changing the volume of the coating. Fig. 6c shows SCA having machined surfaces instead of SLA surfaces.

Figure 7a shows the surface microroughness measured on discs variated by keratin concentration change. Figure 7b shows the microroughness measurement of ZLA coated dental implants.

Figure 8a shows the static contact angle of keratin protection layer at low and high concentrations. Figure 8b shows the static contact angle of low concentration protection layers with and without a water washing pretreatment.

Figure 9 shows the static contact angle affected by EO sterilization or climatic stress cycle.

Figure 10 shows static contact angle of protection layers at low concentration and different storage times.

Figures 11a and 11b show a 3D surface representation from ZLA dental implants roughness measurements in the apex zone.

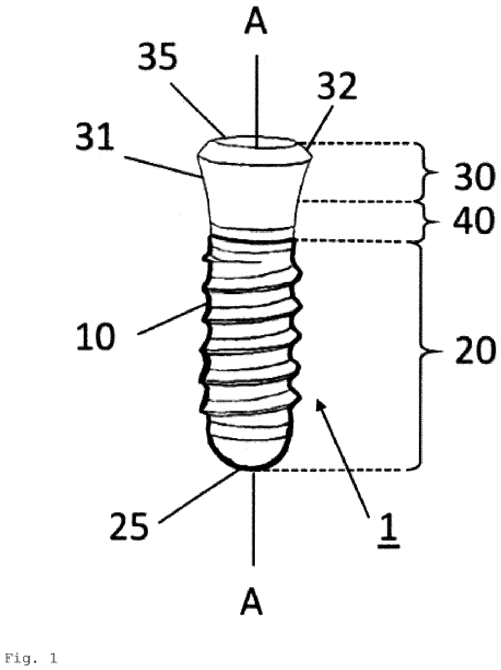

Figure 1 shows an anchoring part 1 of a two-part implant system. Such an anchoring part 1 is made of a ceramic material, preferably of an yttria stabilized zirconia. Said anchoring part 1 is in a cylindrical shape, having an apical end 25 with a body portion 20 and a coronal end 35 with a neck portion 30 intended to receive an abutment, and a transition portion 40 being arranged in axial direction A between the body portion 20 and the neck portion 30. The neck portion 30 includes an unthreaded part 31 which is in coronal direction outwardly tapering and ends in a shoulder portion 32 with an inwardly-tapering surface. The body portion 20 is intended to be directed against the bone tissue in the implanted state, whereas the neck portion 30 is intended to be directed against the soft tissue in the implanted state. The transition portion 40 may be directed towards the soft tissue and the bone tissue depending on the depth to which the implant is screwed or on the tissue reaction. The surface of the body portion 20 has a contact angle of less than 20°, which is preferably entirely covered with the protective layer 10.

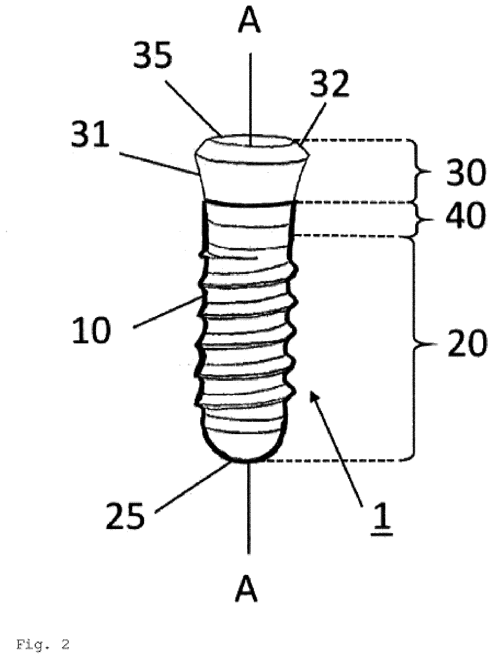

Figure 2 shows another embodiment of the present invention. In contrast to the embodiment of Figure 1, not only the body portion 20 but also the transition portion 40 of the anchoring part is at least partly, preferably entirely coated with said protective layer 10. Preferably, in apical direction, at least 25%, most preferably at least 50% of the circumferential surface area of the transition portion is covered with the protective layer 10. Preferably, also at least a part preferably the entire surface of the transition portion has a contact angle of less than 20°, before covering it with the protective coating 10. However, it is also possible, that only the surface of the body portion of the dental implant has a contact angle of less than 20°, but the protective layer covers both, body portion and transition portion. This allows to ensure that also the hydrophilic surface of the edges is fully protected by the protective layer.

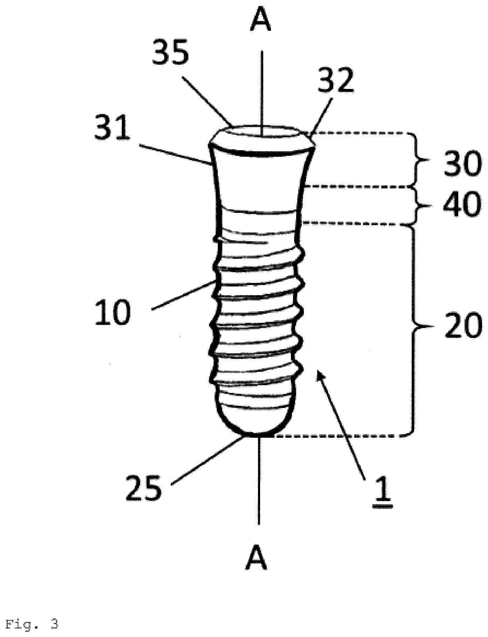

Figure 3 shows another embodiment of the present invention. In contrast to the embodiment of Figure 1, not only the body portion 20 but also the transition portion 40 and the neck portion 30 except for the shoulder portion 32 are completely coated with the protective layer 10. Also at least a part, preferably the entire surface of the transition portion and at least a part of the neck portion has a contact angle of less than 20°, before covering it with the protective layer 10. It could be shown, that a hydrophilic surface not only ensures a good osseointegration but also positively influences the adherence to the soft tissue. However, it is also possible, that only the surface of the body portion of the dental implant and optionally part of the transition portion has a contact angle of less than 20°, but the protective layer covers both, body portion and transition portion.

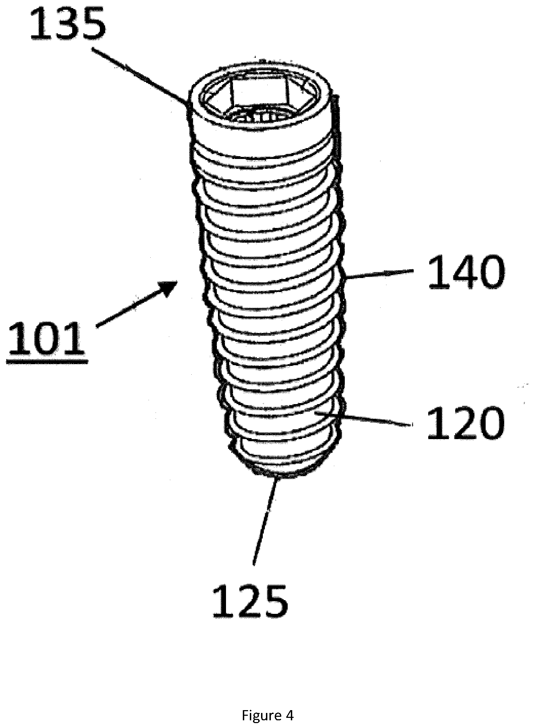

Figure 4 shows an anchoring part 101 of a so-called bone level implant. Such a bone level implant is usually embedded completely in the bone, that is to say to the height of the alveolar crest. Said anchoring part 101 is made of a ceramic material, preferably of an yttria stabilized zirconia. The anchoring part 101 is in a cylindrical shape, having an apical end 125 with a body portion 120 and a coronal end 135 intended to receive an abutment. In contrast to the dental implant shown in figure 1, the anchoring part of a bone level implant has no neck portion. The body portion 120 is intended to be entirely directed against the bone tissue in the implanted state. The surface of the body portion 120 has a contact angle of less than 20°, which is entirely covered with the protective layer 110.

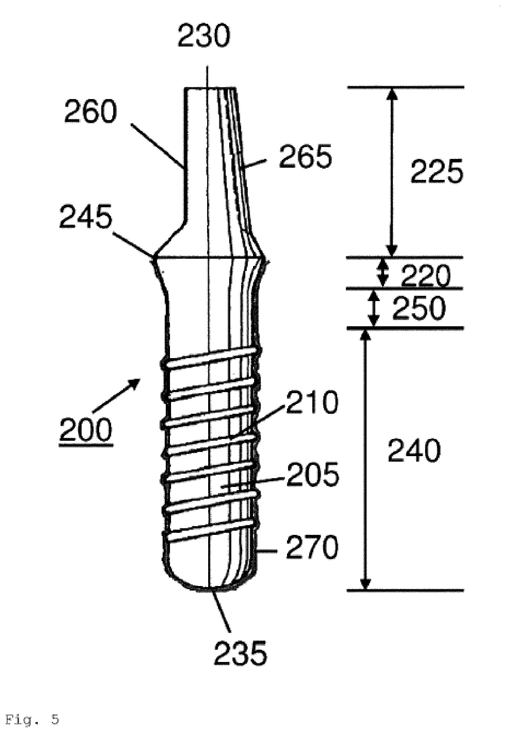

Figure 5 shows a monotype dental implant 200. Said monotype dental implant 200 is made of a ceramic material, preferably of an yttria stabilized zirconia. It comprises an anchoring part 205 having a threaded section 210. The anchoring part 205 at its upper end 215 transitions via a slightly enlarged conical neck portion 220 to the outside into a mounting part 225 being integral therewith and extending within an extension of the longitudinal axis 230 of the threaded section. Said anchoring part 205 is in a cylindrical shape, having an apical end 235 with a body portion 240 and a coronal end 245 with a neck portion 220, and a transition portion 250 being arranged in axial direction A between the body portion 240 and the neck portion 220.

The mounting part 220 has a frusto-conical or a conical shape and may be provided with at least one a flattening 230 at one side thereof.

At the side opposite the at least one flattening 260 there may be a groove 265 within the outer surface that extends from the coronal front surface of the mounting part 225 toward the apical side and ends in a conical section which forms the transition to the conical section of the anchoring part 205. The flattening 260 in combination with the groove 265 located on the opposite side functions to provide a positive a screwing tool which has a plug-in seat matched thereto. Alternatively, the mounting part may be provided with other means for receiving a screwing tool. The body portion 240 is intended to be directed against the bone tissue in the implanted state and the neck portion 220 is intended to direct against the soft tissue, whereas the transition portion 250 may direct against the bone tissue or the soft tissue depending on the patient. The surface of the body portion 240 has a contact angle of less than 20°, which is at least partly, preferably entirely covered with the protective layer 270. Optionally, not only the body portion 240 but also the transition portion 250 of the anchoring part is hydrophilic. Preferably, the transition portion 250 has a contact angle of less than 45°, preferably of less than 20°, which is covered by the protective layer 270. Preferably, at least 25%, most preferably at least 50% of the apical circumferential surface area of the transition portion is covered with said protective layer 250. This allows more flexibility when implanting the implant and ensures that the whole surface, which is intended to be in contact with the bone tissue, is hydrophilic.

## Examples

## Material

The term ZLA within the context of the present invention stands for yttria stabilized zirconia, i.e. 3Y-TZP according to DIN ISO 12677, having a sand blasted (corundum 0.1-0.4 mm, 6 bar) and acid etched surface (for example HF). The used keratin hydrolyzate was Nutrilan ® Keratin LM, BASF (CAS-Nr. 69430-36-0) with a molecular weight of 2000 g/mol. Ker:$MgCl_2$ stands for a Nutrilan ® Keratin LM : $MgCl_2$ ratio of 9:1, whereby the keratin solution had a concentration of 1 mg / ml and the $MgCl_2$ solultion had a concentration of 0.05M.

Within the context of the present invention, "low concentration" stands for about 1 mg/ml (i.e. for example 1 mg Nutrilan ® Keratin LM in 1 ml water which was obtained by adding 870 pl water per 100 ml of Nutrilan Keratin having a concentration of 115 mg/ml). The expression high concentration stands for about 30 mg /ml.

## $O_2$ plasma cleaning (Hydrophilic treateament, HPL treatment)

Samples used for coating experiments were cleaned and stored as follows:
Open the gas bottle and connect the $O_2$ plasma cleaner and the valve to plasma. Press the pump button and wait until the pressure is lower than $8*10^{-2}$ mbar to switch on the gas. Give more gas flow (until $4-5*10^{-1}$ mbar) and wait 5 minutes. Lower the gas flow to $1*10^{-1}$ mbar and press the Generation button. Make sure that the parameters are set to 4 minutes (or two cycles of 2 minutes) and 35W. Turn flow to 0 and switch off the gas. Stop pumping and open the chamber (Ventilation button). Take the samples holders from inside. Place the samples to be cleaned. Start cleaning procedure. Measure the contact angle of 3 samples to make sure that the process was successful (contact angle must be of a value of 0 degrees).

## Coating procedure - discs

The sample was dipped in the dipping solution for 3 minutes on the coating beakers under sonication, placed on a Teflon mesh, placed in a shaker at 150 rpm for three minutes to get a more homogeneous coating and dried. The drying was carried out with recirculating air at 55 °C for 15 minutes in order to dry the coating on the samples.

Storage: the samples were stored in 24 well plates under laminar flow if no EO sterilization was performed.

## Coating procedure - implants

Same procedure as for disks, with the following exceptions: No implants holder was used. They were submerged one by one in the coating solution and taken out with ceramic tweezers. Further, the implants were not placed in a Teflon mesh for trying, but directly in the implants primary package.

## Static contact angle

The experimental procedure was carried out according to DIN 55660-2:2011-12. Usually, the static contact angles were determined using a sessile drop test with ultrapure water (EasyDrop DSA20E, Kruss GmbH). The water droplets with a size of 0.1 $\mu$L for hydrophilic and 0.3 $\mu$L for hydrophobic discs of 5 mm diameter were dosed using an automated unit. Values for contact angles were calculated by fitting a circular segment function to the contour of the droplet placed on the surface. Contact angles were determined for two different cases: (a) without washing the protection layer away and (b) after rinsing the samples with UPw for 15 seconds followed by blow drying in a stream of ar in order to remove the coating.

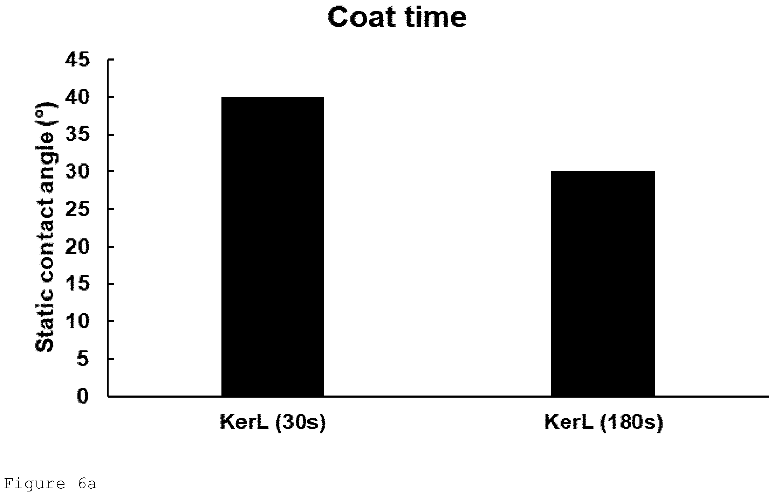

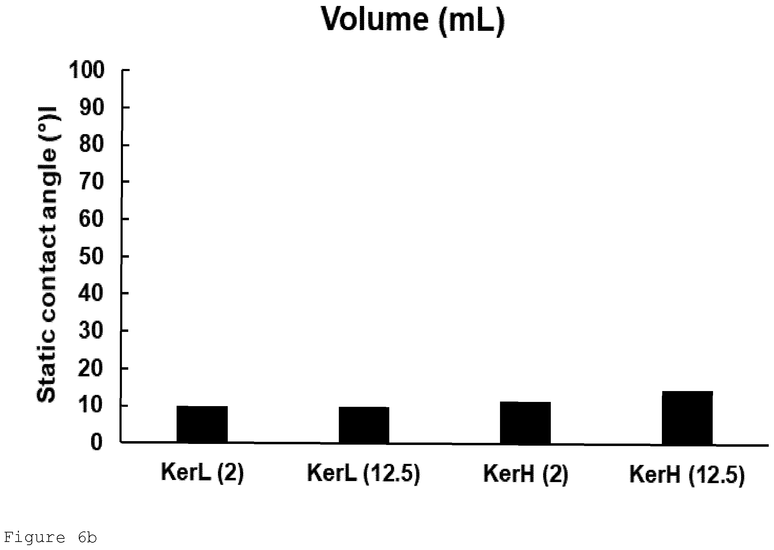

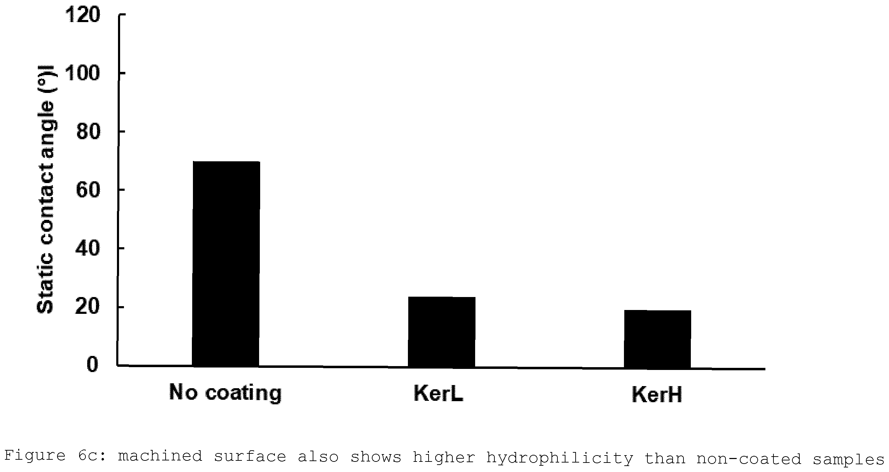

As shown in Figures 6a to 6c, the protective layer according to the present invention can be produced in a reproducible manner. It was shown that neither the coating duration (30 seconds vs 180 seconds; Fig. 6a) nor the volume of the coating solution (2 ml vs 12.5 ml; Fig. 6b) has a significant influence on the static contact angle (SCA). In addition, it was shown that other surfaces, such as machined instead of SLA, showed improved wettability, and therefore higher hydrophilicity than the non-coated machined surfaces. (Fig. Fig. 6c).

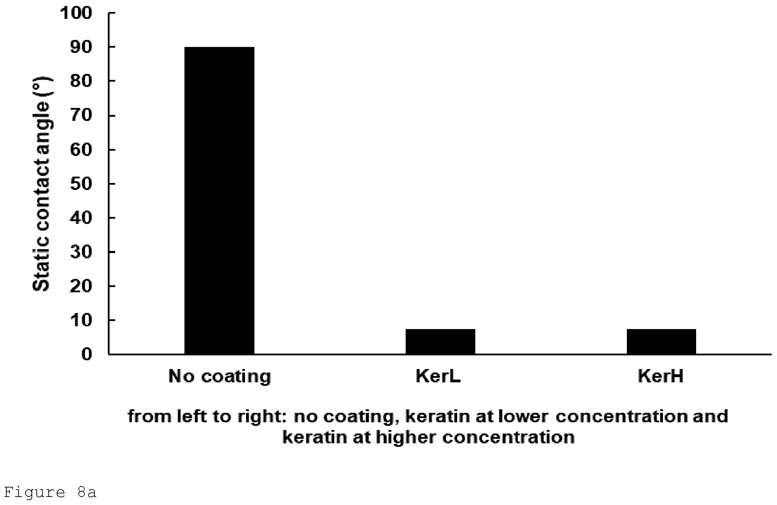

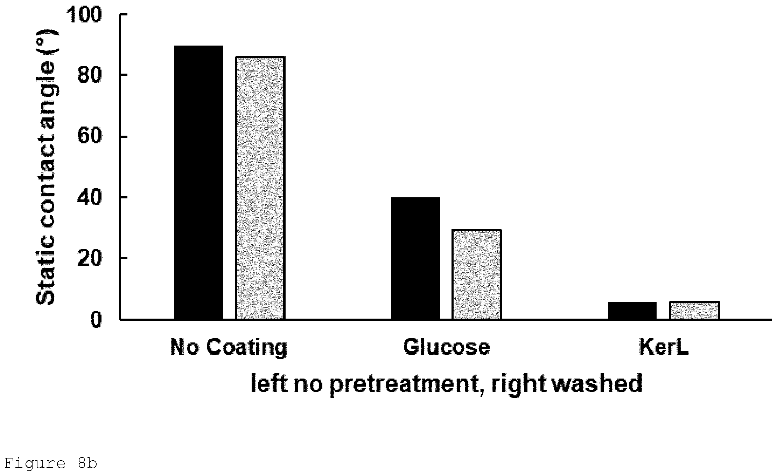

Further, in Figure 8a it is shown that an increase of the keratin hydrolyzate concentration (1 mg/mL for KerL vs. 30 mg/mL for KerH) does not change the hydrophilicity (no EO sterilization, n=1 measurement, t=1 week, protective layer not washed). As shown in Figure 8b other protection layers (such as glucose) at low concentration (1mg/mL) cannot provide with the desired contact angles under 25°.

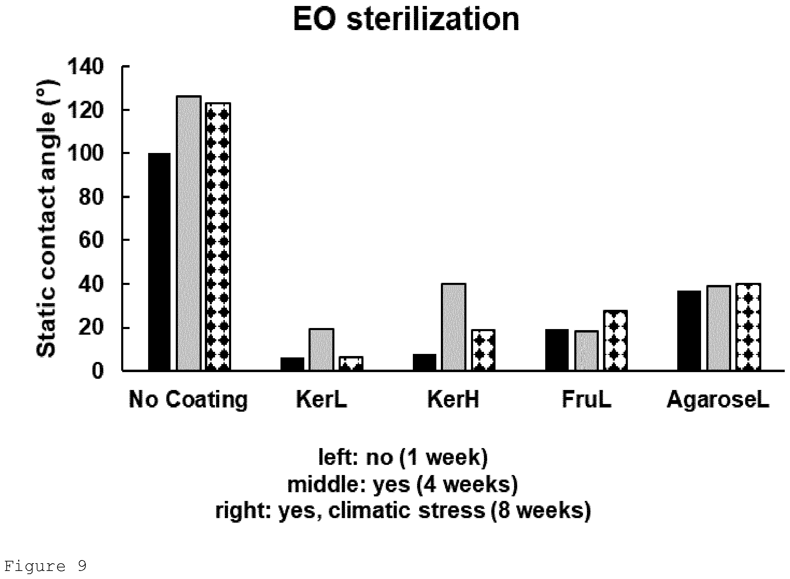

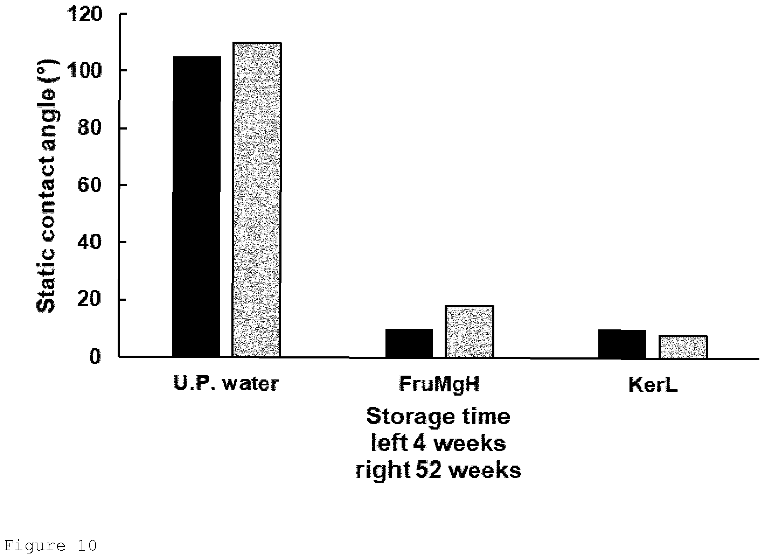

Among the investigated protective layers at low concentrations only the discs coated with low-concentration keratin hydrolyzate could fulfil the criteria of having a contact angle below 25° without additional washing step performed by the clinical, ensuring sufficient hydrophilicity of the surface at the time of implantation (Figure 9; static contact angle of protective layers at low concentration (1mg/mL) and keratin layer at low and high concentrations (1mg/mL vs. 10mg/mL); EO sterilization ; n=2 samples ; t=4 weeks). If higher concentrations of sugary protection layers were used, such as fructose at 30mg/mL, lower contact angles were also seen, even though longer storage times involved showed possible sugary layers deteriorement (Figure 10; static contact angle of protective layers at 4 and 52 weeks storage time; EO sterilization; n=3 samples; PL not removed).

## Dynamic contact angle

Following the Wilhelmy plate method by means of a tensiometer (Lauda TE 3, Lauda Dr. R. Wobser GmbH & Co. KG), when a solid is submerged into a liquid and afterwards extracted from the liquid, the surface tension can be calculated from the necessary force that is needed for that specific solid.

The dynamic contact angle performed on coated ZLA implants showed that a coating with keratin hydrolyzate resulted in superhydrophilic implant surfaces, significantly outperforming their counterparts.

## Thickness estimation by microbalance

Effective microroughness values calculated using the 3D roughness values to be determined according to relevant procedure defined by Straumann Research with a low-pass Gaussian filter at 30 $\mu$m cut-off. Values of non-coated samples are used for all calculations, since that is the available surface for a coating.

$$Thickness = \frac{\Delta weight}{Area * density} * \left(\frac{1 + eff. \, microroughness}{100}\right)$$

Samples preparation before weight measurement

The samples have to be dried before weight measurement (15 minutes in the oven at 55°C; afterwards 110°C for 40 minutes).

The weight difference of the discs before and after coating resulted in the following thickness estimation:

| Protective layer | Estimated thickness (nm) | Density (mg/mm$^3$) |
|---|---|---|
| FruMgH | 1349.54 | 1.03 |
| KerMgL | 66.40 | 1.06 |
| GluMgH | 615.54 | 1.01 |
| DexMgH | 618.50 | 1.1 |

## Surface microroughness

Since the samples have a specific micro surface roughness after sandblasting and acid etching, depending on the coating, the specific roughness parameters will change. These parameters are defined by the ISO 25178:
Ssk (AU): Skewness represents the symmetry of the surface heights in relation to the mean plane. If the parameter is larger than 0, peaks are predominant in front of valleys, while if the parameter is smaller than 0, valleys are predominant.

Sa ($\mu$m) and Sz ($\mu$m): Arithmetic mean height and maximum height represent a measure of the surface texture, containing information about the peaks and valleys, as well as information about the spacing in among those surface characteristics.

Sdr ($\mu$m): Developed interfacial area ratio represents the percentage of additional surface area that has to be added in relation to a complete flat surface without a texture. Before analyzing the samples, they should be shortly rinsed under Argon gas in order to remove dust from the environment.

Parameters used: (objective lens: 20x/0.45 - Mplan FL N ; horizontal step distance: 0.22 $\mu$m; illumination: 70% exposure time / algorithm / quality: 28.5 ms / quick / max; gain: 1.5 dB).

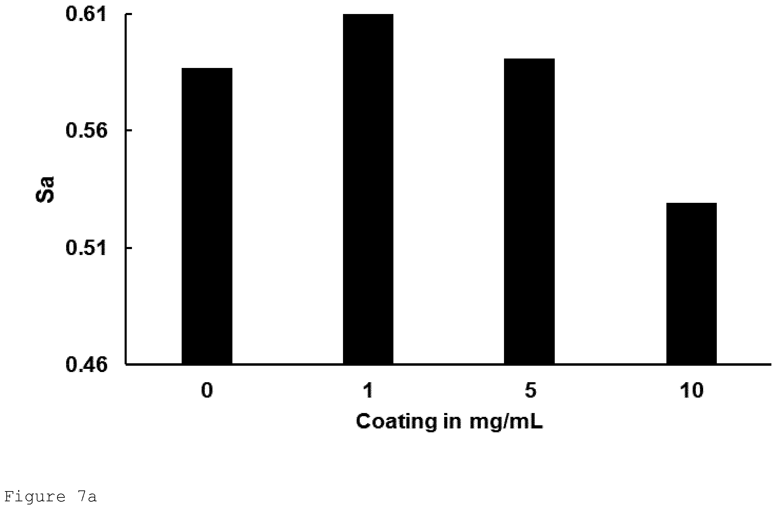

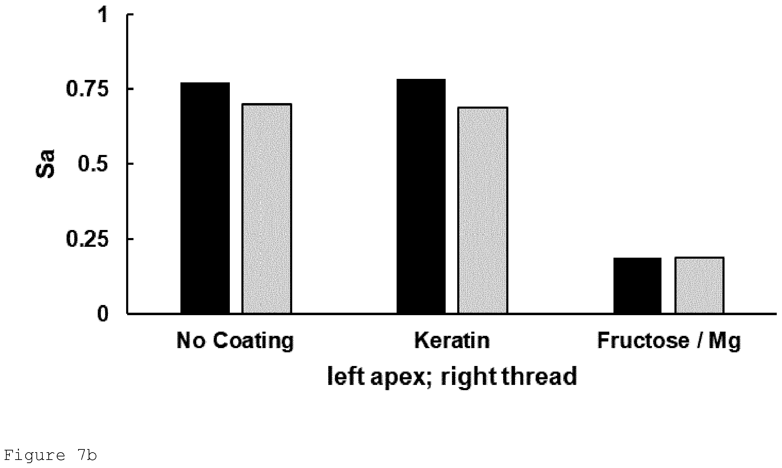

In Figure 7 the surface roughness Sa is shown with respect to the the keratin layer and the concentration thereof. A higher keratin concentration results in a decrease of Sa pointing to a flatter surface. In addition, Figure 7b shows the effect of keratin and fructose on the surface roughness for implant materials. In the absence of any coating, Sa is mainly in the range of 0.4 to 0.6 $\mu$m. Introduction of keratin results in a maintance of the surface microroughness, while introduction of fructose layer results in a typical out of range SLA microroughness.

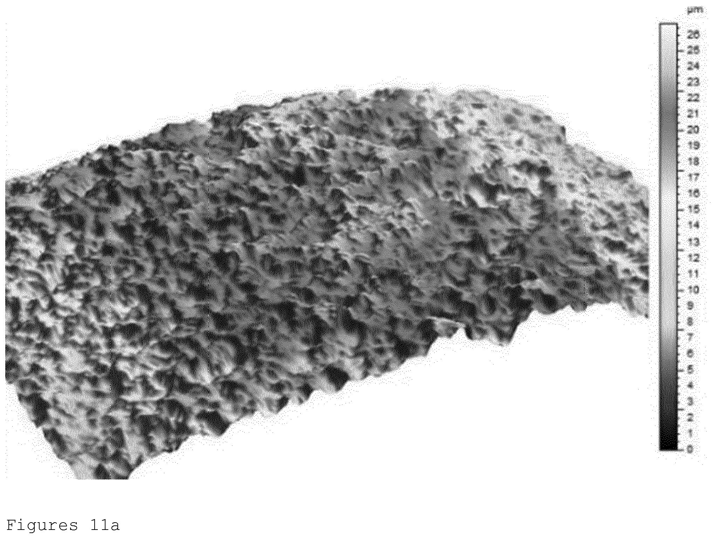

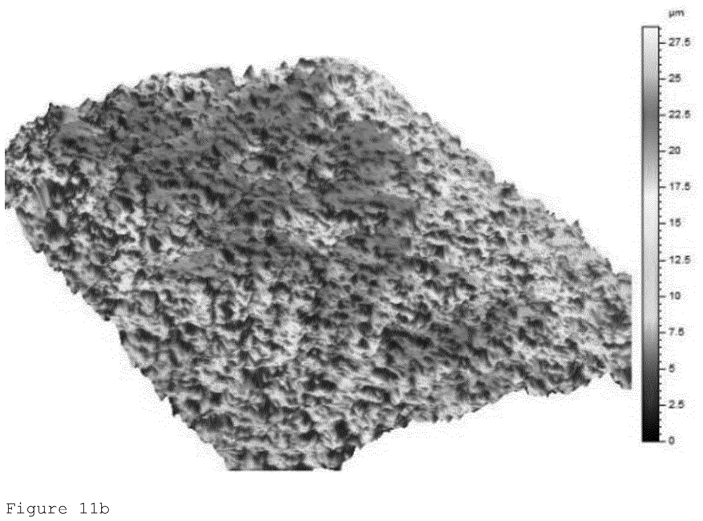

Microroughness measurements of the coated implants, specifically Sa values, were compared between the apex (tip of the implant) and the 6th thread starting from the apex of the implant (Figure 7b). In a qualitative examination, differences between the uncoated surface (Figure 11a) and the surface coated with the protective layer (Figure 11b) show that they both have a lot of hills and valleys at different heights.

**Packaging climatic stress cycle applied to Y-TZP materials**

Table 1. Climatic conditions (with tolerance limits) to be applied

| Step | Cycle | Temp (°C) | Time (h) | Relative Humidity (RH,%) |
|---|---|---|---|---|
| 1 | Ambient | 23 | 6 | 30-80 |
| 2 | Hot-wet | 50 | 16 | 95 |
| 3 | Ambient | 23 | 1 | 50 |
| 4 | Cold | -33 | 16 | Not controlled |
| 5 | Ambient | 23 | 1 | 50 |
| 6 | Hot-dry | 60 | 16 | 10 |

In order to check if the protective layers would be damaged under critical conditions during their storage, the cycle in Table 1 was applied after EO sterilization and the contact angle of the samples was measured and compared to the contact angle of the samples without going under this stress cycle.

As shown in Figure 9, overall, no effect due to climatic stress could be detected (tendency of the contact angle to increase, i.e. more hydrophobic).

**Kinetic aging test**

Aging parameters are established according to ASTM F1980 - 16 - Accelerated aging of sterile barrier systems for medical devices.

At $2\theta=30°$, the higher intensity for the tetragonal phase was measured, with a depth of approximately 9.4$\mu$m. The maximum depth penetration in the sample was about 20um, depending on the specific sample and if the mentioned was coated or not. The measure was performed from 10 to 40 incident ray degree

**Sterility test**

In order to test if the coatings provide antimicrobial activity, each sample was incubated in Lysogeny broth with E. Coli bacteria. E.Coli was pre-cultured one day before the infection start. At the time of the infection, a dissolution of the bacteria in fresh media to reach a concentration of 1*106 bacteria/mL were incubated (to test the amount of bacteria, Optical Density (OD) at 600nm was measured and adjusted to 0.001). A control group was added, where no sample was added. From this control, the number of bacteria after 1 day is measured and compared to the samples of interest.

The bioburdent test consists of 7 days incubation of the samples in a LB media. Afterwards, OD-values of medium are taken to quantify the bacterial growth. If bacteria is observed, plating for CFU determination is needed.

Each protective layer was analyzed with three different replicates.

Sterility test in BBF SteriXpert

All samples were EO sterilized before analysis. The PLs were not removed, but cultivated in the media, so that sterilization was tested on the surface of the discs and in the dissolved components of the protective layers.

A caso broth was incubated at 30°C in aerobic conditions during 14 days. Different media was used for each sample. Afterwards, visual analysis was performed to see if there were bacterial growth in the caso, according to ISO 11737-2. The analyzed microorganisms were the following:

- Aerobic spore forming bacteria
- Staphylococci
- Micrococci
- Moulds
- Yeasts

In this case, one replicate was used for each PL.

## Blood wettability of implants

As a proof of concept, the implants were dipped into blood. The blood was obtained fresh, and the implants were dipped into the blood for 10 seconds at the speed of 0.166 mm/second. After keeping for 6 minutes the implants in the blood (at 1.66 mm depth), the uncoated implant did not wet with blood media. Keratin layers quickly adsorbed blood, which resulted in an increasing quick red coloration of the threads from the apex upwards (after one minute it was already at the maximum blood adsorption). Blood wettability of implants after 6 minutes made at the DCA instrument (at 1.66mm depth from the first contact to the apex)(a) NoCoating (b)Ker].

## Claims

1. Dental implant (1) comprising an implant surface having at least partially a contact angle of less than 20°, said implant surface being at least partially covered with a protective layer (10) comprising a keratin hydrolyzate wherein said protective layer (10) has a water content of less than 10% by weight.

2. Dental implant (1) according to claim 1, wherein the keratin hydrolyzate has a mean molecular weight of less than 20'000 Da, preferably less than 10'000 Da, most preferably less than 5'000 Da.

3. Dental implant (1) according to any of the preceding claims, **characterized in that** the keratin hydrolyzate layer has a thickness of 30 to 200 nm.

4. Dental implant (1) according to claim 3, **characterized in that** the keratin hydrolyzate layer has a thickness of 30 to 90 nm.

5. Dental implant (1) according to any of the preceding claims, **characterized in that** the protective layer (10) is essentially free from any salt.

6. Dental implant (1) according to any of claims 1 to 4, **characterized in that** the protective layer (10) additionally comprises a salt, preferably a bivalent salt, and most preferably $MgCl_2$.

7. Dental implant (1) according to any of claims 1 to 6, **characterized in that** the dental implant is made of ceramic, preferably of an yttria stabilized zirconia ceramic.

8. Dental implant (1) according to any of the preceding claims, **characterized in that** the surface is provided with surface roughness Sa in a range between 2 and 10 μm, preferably between 2 and 5 μm.

9. Dental implant (1) according to any of claims 1 to 7, **characterized in that** said implant has a machined surface.

10. Dental implant (1) according to any of the preceding claims, wherein the protective layer (10) has a water content of less than 1% by weight.

11. Method for preparing a dental implant (1) to any of the preceding claims comprising at least the following steps:

   a) providing the surface of a dental implant (1) having at least partially contact angle of less than 20°,
   b) covering at least partially the surface of said dental implant with a solution or suspension comprising the keratin hydrolyzate, and
   c) drying the dental implant in order to obtain a protective layer (10).

**12.** Method according to claim 11, **characterized in that** in step b) the surface is covered by dipping the dental implant into an aqueous solution or suspension.

**13.** Method according to any of claims 11 to 12 **characterized in that** in step c) the water is removed by microwave treatment, by airstream or by drying in a convection oven or a vacuum oven.

**14.** Method according to any of claims 11 to 13, **characterized in that** aqueous solution or suspension comprises the hydrolyzed keratin in a concentration of 0.1 to 10% (w/v), preferably 1 to 5% (w/v).

**15.** Method according to any of claims 11 to 14, **characterized in that** the dental implant covered with the protective layer (10) is sterilized in a further step by ethylene oxide.

**Patentansprüche**

**1.** Zahnimplantat (1), enthaltend eine Implantatoberfläche, die zumindest teilweise einen Kontaktwinkel von weniger als 20° aufweist, wobei die Implantatoberfläche zumindest teilweise mit einer Schutzschicht (10) bedeckt ist, die ein Keratinhydrolysat enthält, wobei die Schutzschicht (10) einen Wassergehalt von weniger als 10 Gew.-% aufweist.

**2.** Zahnimplantat (1) gemäß Anspruch 1, wobei das Keratinhydrolysat ein mittleres Molekulargewicht von weniger als 20'000 Da, vorzugsweise weniger als 10'000 Da, am meisten bevorzugt weniger als 5'000 Da, aufweist.

**3.** Zahnimplantat (1) nach einem der vorhergehenden Ansprüche, **dadurch gekennzeichnet, dass** die Keratinhydrolysatschicht eine Dicke von 30 bis 200 nm aufweist.

**4.** Zahnimplantat (1) nach Anspruch 3, **dadurch gekennzeichnet, dass** die Keratinhydrolysatschicht eine Dicke von 30 bis 90 nm aufweist.

**5.** Zahnimplantat (1) gemäß einem der vorhergehenden Ansprüche, **dadurch gekennzeichnet, dass** die Schutzschicht (10) im Wesentlichen frei von jeglichem Salz ist.

**6.** Zahnimplantat (1) nach einem der Ansprüche 1 bis 4, **dadurch gekennzeichnet, dass** die Schutzschicht (10) zusätzlich ein Salz, vorzugsweise ein zweiwertiges Salz, und am meisten bevorzugt MgCl, enthält.

**7.** Zahnimplantat (1) gemäß einem der Ansprüche 1 bis 6, **dadurch gekennzeichnet, dass** das Zahnimplantat aus Keramik besteht, vorzugsweise aus einer Yttriumoxidstabilisierten Zirkonoxidkeramik.

**8.** Zahnimplantat (1) gemäß einem der vorhergehenden Ansprüche, **dadurch gekennzeichnet, dass** die Oberfläche eine Oberflächenrauheit Sa in einem Bereich zwischen 2 und 10 $\mu$m, vorzugsweise zwischen 2 und 5 $\mu$m, aufweist.

**9.** Zahnimplantat (1) gemäß einem der Ansprüche 1 bis 7, **dadurch gekennzeichnet, dass** das Implantat eine bearbeitete Oberfläche aufweist.

**10.** Zahnimplantat (1) gemäß einem der vorhergehenden Ansprüche, wobei die Schutzschicht (10) einen Wassergehalt von weniger als 1 Gew.-% aufweist.

**11.** Verfahren zur Vorbereitung eines Zahnimplantats (1) gemäß einem der vorhergehenden Ansprüche, enthaltend mindestens die folgenden Schritte:

a. Bereitstellen der Oberfläche eines Zahnimplantats (1) mit einem Kontaktwinkel von mindestens teilweise weniger als 20°,
b. mindestens teilweises Bedecken der Oberfläche des Zahnimplantats mit einer Lösung oder Suspension, enthaltend das Keratinhydrolysat, und
c. Trocknen des Zahnimplantats, um eine Schutzschicht (10) zu erhalten.

**12.** Verfahren gemäß Anspruch 11, **dadurch gekennzeichnet, dass** in Schritt b) die Oberfläche durch Eintauchen des Zahnimplantats in eine wässrige Lösung oder Suspension bedeckt wird.

**13.** Verfahren gemäß einem der Ansprüche 11 bis 12, **dadurch gekennzeichnet, dass** in Schritt c) das Wasser durch Mikrowellenbehandlung, durch einen Luftstrom, oder durch Trocknen in einem Konvektionsofen oder einem Vakuumofen entfernt wird.

**14.** Verfahren gemäß einem der Ansprüche 11 bis 13, **dadurch gekennzeichnet, dass** die wässrige Lösung oder Suspension das hydrolysierte Keratin in einer Konzentration von 0,1 bis 10% (Gew./Vol.), vorzugsweise 1 bis 5% (Gew./Vol.), enthält.

**15.** Verfahren gemäß einem der Ansprüche 11 bis 14, **dadurch gekennzeichnet, dass** das mit der Schutzschicht (10) überzogene Zahnimplantat in einem weiteren Schritt mit Ethylenoxid sterilisiert wird.

**Revendications**

**1.** Implant dentaire (1) comprenant une surface d'implant ayant au moins partiellement un angle de contact inférieur à 20°, ladite surface d'implant étant au moins partiellement recouverte d'une couche protectrice (10) comprenant un hydrolysat de kératine, dans lequel ladite couche protectrice (10) a une teneur en eau inférieure à 10 % en poids.

**2.** Implant dentaire (1) selon la revendication 1, dans lequel l'hydrolysat de kératine a un poids moléculaire moyen inférieur à 20 000 Da, de préférence inférieur à 10 000 Da, et de préférence encore inférieur à 5 000 Da.

**3.** Implant dentaire (1) selon l'une quelconque des revendications précédentes, **caractérisé en ce que** la couche d'hydrolysat de kératine a une épaisseur de 30 à 200 nm.

**4.** Implant dentaire (1) selon la revendication 3, **caractérisé en ce que** la couche d'hydrolysat de kératine a une épaisseur de 30 à 90 nm.

**5.** Implant dentaire (1) selon l'une quelconque des revendications précédentes, **caractérisé en ce que** la couche protectrice (10) est essentiellement exempte de tout sel.

**6.** Implant dentaire (1) selon l'une quelconque des revendications 1 4, **caractérisé en ce que** la couche protectrice (10) comprend en outre un sel, de préférence un sel bivalent, et plus préférablement de $MgCl_2$.

**7.** Implant dentaire (1) selon l'une quelconque des revendications 1 à 6, **caractérisé en ce que** l'implant dentaire est en céramique, de préférence en céramique de zircone stabilisée à l'yttria.

**8.** Implant dentaire (1) selon l'une quelconque des revendications précédentes, **caractérisé en ce que** la surface présente une rugosité de surface Sa comprise entre 2 et 10 $\mu$m, de préférence entre 2 et 5 $\mu$m.

**9.** Implant dentaire (1) selon l'une quelconque des revendications 1 à 7, **caractérisé en ce que** ledit implant a une surface usinée.

**10.** Implant dentaire (1) selon l'une quelconque des revendications précédentes, dans lequel la couche protectrice (10) a une teneur en eau inférieure à 1 % en poids.

**11.** Procédé de préparation d'un implant dentaire (1) selon l'une quelconque des revendications précédentes, comprenant au moins les étapes suivantes :

a) fournir la surface d'un implant dentaire (1) ayant au moins partiellement un angle de contact inférieur à 20°,
b) recouvrir au moins partiellement la surface dudit implant dentaire d'une solution ou d'une suspension comprenant l'hydrolysat de kératine, et
c) sécher l'implant dentaire afin d'obtenir une couche protectrice (10).

**12.** Procédé selon la revendication 11, **caractérisé en ce que** dans l'étape b), la surface est recouverte en plongeant l'implant dentaire dans une solution ou suspension aqueuse.

**13.** Procédé selon l'une quelconque des revendications 11 à 12, **caractérisé en ce que** dans l'étape c), l'eau est éliminée par traitement aux micro-ondes, par courant d'air ou par séchage dans un four à convection ou un four à vide.

**14.** Procédé selon l'une quelconque des revendications 11 à 13, **caractérisé en ce que** la solution ou suspension aqueuse comprend la kératine hydrolysée à une concentration de 0,1 à 10 % (p/v), de préférence de 1 à 5 % (p/v).

**15.** Procédé selon l'une quelconque des revendications 11 à 14, **caractérisé en ce que** l'implant dentaire recouvert de la couche protectrice (10) est stérilisé dans une étape ultérieure par de l'oxyde d'éthylène.

Fig. 1

Fig. 2

Fig. 3

Figure 4

Fig. 5

Figure 6a

Figure 6b

Figure 6c: machined surface also shows higher hydrophilicity than non-coated samples

Figure 7a

Figure 7b

from left to right: no coating, keratin at lower concentration and keratin at higher concentration

Figure 8a

Figure 8b

# EO sterilization

left: no (1 week)
middle: yes (4 weeks)
right: yes, climatic stress (8 weeks)

Figure 9

Figure 10

EP 4 236 861 B1

Figures 11a

Figure 11b

## REFERENCES CITED IN THE DESCRIPTION

**Patent documents cited in the description**

- EP 0388576 A
- WO 0044305 A
- US 2014172028 A
- WO 2018189185 A
- WO 2008098976 A
- US 20090132048 A
- WO 03030957 A
- US 2015367043 A1
- US 2007202144 A1
- EP 1982670 A
- EP 1982671 A

**Non-patent literature cited in the description**

- **CAMPBELL et al.** The Effect of a Keratin Hydrogel Coating on Osseointegration: A Histological Comparison of Coated and Non-coated Dental Titanium Implants in an Ovine Model. *JOURNAL OF MAXILLOFACIAL AND ORAL SURGERY*, 01 February 2013, vol. 13 (2), 159-164
- Material properties and cell compatibility of poly ([gamma]-glutamic acid)-keratin hydrogels. **BAJESTANI et al.** INTERNATIONAL JOURNAL OF BIOLOGICAL MACROMOLECULES. ELSEVIER BV, 14 October 2019, vol. 142, 790-802
- Current research on the blends of natural and synthetic polymers as new biomaterials: Review. **SIONKOWSKA et al.** PROGRESS IN POLYMER SCIENCE. PERGAMON PRESS, 04 May 2011, vol. 36, 1254-1276
- **KREJCI et al.** preparation and characterization of keratin hydrolyzates. *Mathematical Methods and Techniques in Engineering and Environmental Science*, ISBN 978-1-61804-046-6
- *CHEMICAL ABSTRACTS*, 69430-36-0